(19) Europäisches Patentamt
European Patent Office
Office européen des brevets

(11) EP 1 250 915 A1

(12) # EUROPÄISCHE PATENTANMELDUNG

(43) Veröffentlichungstag:
**23.10.2002 Patentblatt 2002/43**

(51) Int Cl.[7]: **A61K 7/48**

(21) Anmeldenummer: **01109642.7**

(22) Anmeldetag: **19.04.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder: **Beiersdorf Aktiengesellschaft
20245 Hamburg (DE)**

(72) Erfinder:
- **Bleckmann, Andreas
22926 Ahrensburg (DE)**
- **Syskowski, Boris
22303 Hamburg (DE)**
- **von Thaden, Stefanie
20255 Hamburg (DE)**
- **Hamer, Gunhild
22455 Hamburg (DE)**

(54) **Kosmetische und dermatologische Zubereitungen, enthaltend erhöhte Elektrolytkonzentrationen**

(57) Kosmetische und dermatologische Emulsionen mit mindestens einer wässrigen Phase,
die

a) mindestens einen hydrophilen Emulgator A, gewählt aus der Gruppe der ethoxylierten Emulgatoren mit einem Ethoxylierungsgrad von 20 bis 100,
b) mindestens einen hydrophoben Emulgator B, gewählt aus der Gruppe, die gebildet wird aus ethoxylierten Emulgatoren mit einem Ethoxylierungsgrad von 1 bis 5 und Stearinsäurederivaten, sowie
c) mindestens einen Fettalkohol, gewählt aus der Gruppe der verzweigten oder unverzweigten, gesättigten oder ungesättigten, aliphatischen Fettalkohole mit einer Kettenlänge von $C_{14}$ bis $C_{22}$, enthalten,

wobei das Verhältnis von Emulgator A zu Emulgator B zu Fettalkohol aus dem Bereich 2 : 3 : 5 bis 2 : 5 : 3 gewählt wird.

EP 1 250 915 A1

**Beschreibung**

Die vorliegende Erfindung betrifft kosmetische und dermatologische Emulsionen, insbesondere hautpflegende kosmetische und dermatologische Emulsionen. In einer vorteilhaften Ausführungsform betrifft die vorliegende Erfindung eine Anwendung, welche es erlaubt, die Stabilität von elektrolythaltigen Zubereitungen, insbesondere Emulsionen, bevorzugt von O/W-Emulsionen, zu steigern.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muss deshalb ununterbrochen erneuert werden.

Ein heute in der Fachwelt weitverbreitetes Hautmodell fasst das Stratum corneum als Zwei-Komponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluss auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel neben ausgewogenen Lipidabmischungen und Wasser auch wasserbindende Substanzen, um die flüssigkristalline Organisation der Interzellularlipide des Stratum Corneums zu unterstützen und so die Barriereeigenschaften der Hornschicht zu verbessern.

Dabei ist neben der chemischen Zusammensetzung auch das physikalische Verhalten dieser Substanzen von Bedeutung. So ist z. B. die Entwicklung von sehr gut bioverträglichen Emulgatoren bzw. Tensiden wünschenswert. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz). chenden Produkten auf die gesetzlichen Bestimmungen der Bundesrepublik Deutschland verwiesen (z.B. Kosmetikverordnung, Lebensmittel- und Arzneimittelgesetz).

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren in Form feinster Tröpfchen ineinander verteilt werden. Liegt die Ölphase fein verteilt in der Wasserphase vor, so handelt es sich um eine Öl-in-Wasser-Emulsion (O/W-Emulsion, z. B. Milch). Der Grundcharakter einer O/W-Emulsion ist durch das Wasser geprägt, d. h. sie wirkt weniger fettend auf der Haut, ist eher mattierend und zieht schneller in die Haut ein als eine W/O-Emulsion.

Obwohl Emulsionen vom thermodynamischen Standpunkt aus betrachtet instabile Systeme sind, gelingt es, kosmetische Emulsionen von jahrelanger Stabilität herzustellen. Eine Emulsion wird als stabil bezeichnet, wenn sich über einen vorgegebenen Zeitraum hinweg keine messbaren zeitlichen und örtlichen Änderungen der Tropfengrößenverteilung feststellen lassen.

Die Stabilität bzw. Instabilität von Emulsionen hängt von verschiedenen Faktoren ab. Zum einen neigt beispielsweise die Wasserphase einer W/O-Emulsion, da Wasser- und Ölphase unterschiedliche Dichten haben, zur Sedimentation. Die Ölphase einer O/W-Emulsion hat dementsprechend eine Tendenz zum Aufrahmen.

Ferner kann es aufgrund der Anziehungskräfte zwischen den feinverteilten Tröpfchen der dispersen Phase zu einer Tropfenaggregation kommen, wobei die einzelnen Tröpfchen eines Aggregates zunächst durch einen dünnen Film kontinuierlicher Phase voneinander getrennt bleiben. Allerdings können die sich berührenden Tröpfchen auch

zusammenfließen, was zu einer echten Änderung der Tropfengrößenverteilung führt, die nur durch Energiezufuhr wieder verändert werden kann. Dieser Vorgang wird als Koaleszenz bezeichnet. Der Prozess der Koaleszenz läuft umso langsamer ab, je viskoser die äußere Phase der Emulsion ist.

Die beschriebenen Vorgänge können einzeln oder zusammen ablaufen. Oft initiiert oder verstärkt ein Vorgang den anderen. So wird z. B. durch die Bildung von Aggregaten in O/W-Emulsionen das Aufrahmen der Ölphase beschleunigt. Geht der disperse Zustand einer Emulsion teilweise oder auch ganz verloren, so trennen sich die beiden Phasen, und man spricht vom Brechen der Emulsion.

Zur Stabilisierung von Emulsionen über einen längeren Zeitraum hinweg werden dementsprechend Hilfsmittel benötigt, die das Entmischen der beiden Phasen unterbinden, mindestens aber so lange verzögern, bis die Emulsion ihre Bestimmung erfüllt hat.

Diese Hilfsmittel sollen zum einen die Grenzfläche stabilisieren, indem sie verhindern, daß die Tröpfchen der dispersen Phase zusammenfließen. Im Idealfall bewirken diese Stoffe darüber hinaus eine Abstoßung der Tröpfchen, welche verhindert, daß sich diese annähern, so daß eine Zusammenballung (Aggregatbildung) vermieden werden kann. Zum anderen werden Hilfsstoffe dazu eingesetzt, dem Aufrahmen bzw. Sedimentieren der Phasen entgegenzuwirken.

Emulgatoren sind grenzflächenaktive Substanzen, die in der Lage sind, die Grenzflächenspannung zwischen Öl- und Wasserphase zu vermindern, indem sie sich bevorzugt an der Grenzfläche zwischen diesen beiden anlagern. Darüber hinaus stabilisieren Emulgatoren auch durch Ausbildung von Grenzflächenfilmen und damit "physikalischen" Barrieren, wodurch die Aggregatbildung und die Koaleszenz der emulgierten Teilchen verhindert wird.

Eine Möglichkeit, Emulsionen zu stabilisieren, ist nach dem oben gesagten, die Viskosität der äußeren Phase zu erhöhen.

Die Viskosität der äußeren Phase lässt sich beispielsweise durch Zugabe von Verdikkungsmitteln erhöhen, welche z. B. Gele und/oder lamellare Flüssigkristalle ausbilden. Auch Emulgatoren sind im Prinzip in der Lage, durch die Bildung von Emulgatorgelnetzwerken die Viskosität einer Flüssigkeit zu erhöhen. Allerdings wird hierfür eine vergleichsweise hohe Menge an Emulgator benötigt, da Gelnetzwerke erst dann gebildet werden, wenn die gesamte Grenzfläche zwischen den Phasen mit Emulgatormolekülen belegt ist.

Natürlich ist dem Fachmann eine Vielzahl von Möglichkeiten bekannt, stabile O/W-Zubereitungen zur kosmetischen oder dermatologischen Anwendung zu formulieren, beispielsweise in Form von Cremes und Salben oder als Lotionen und Milche. Dabei kommt es neben der Wahl des "richtigen" Emulgators bzw. Emulgatorsystems insbesondere auch auf die weitere Zusammensetzung der Zubereitung an.

O/W-Emulsione werden in der Regel durch Verdickungsmittel, welche die Viskosität der wässrigen Phase erhöhen, zusätzlich stabilisiert. Hierzu eignen sich beispielsweise Polyacrylate (Carbomer) und weitere organische Verdickungsmittel.

Ein Nachteil von O/W-Emulsionen ist ihre Empfindlichkeit gegenüber Elektrolyten. Elektrolyte destabilisieren die Gel-Netzwerk-Struktur von Öl-in-Wasser Emulsionen, und als Folge kommt es häufig zu Emulsionsinstabilitäten wie z. B. Wasser- oder Ölabscheidung.

Es ist aber häufig wünschenswert, bestimmte Elektrolyte, wie beispielsweise wasserlösliche UV-Filter, einzusetzen, um deren sonstige physikalische, chemische bzw. physiologische Eigenschaften nutzen zu können. Zwar lässt sich in vielen Fällen durch geeignete Wahl des Emulgatorsystems in gewissem Maß Abhilfe schaffen, es treten dann aber ebenso oft andere Nachteile auf.

Die angesprochenen Nachteile können beispielsweise darin liegen, daß vergleichsweise große Mengen an einem oder mehreren Emulgatoren erforderlich sind (z. B. 3 Gew.-% oder mehr).

Zwar existiert eine gewisse Auswahl an besonders milden Emulgatoren. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

Aus dem oben gesagten geht hervor, daß die Formulierung von stabilen und hautverträglichen Öl-in-Wasser Emulsionen, welche einerseits einen sehr hohen Elektrolytgehalt (d. h. mit einer aktiven Einsatzkonzentration von mehr als 5 Gew.-%, bezogen auf das Gesamtgewicht der Zubereitungen), andererseits aber auch gute sensorische Eigenschaften zeigen, derzeit nach wie vor sehr schwierig ist.

Was unter "aktiver Einsatzkonzentration" bzw. "Aktivgehalt" ist im Sinne der vorliegenden Erfindung zu verstehen ist, soll das folgende Beispiel verdeutlichen: Liegt ein Rohstoff z. B. in Form einer 50%-igen Lösung vor (beispielsweise weil er nur in dieser Form auf dem Markt erhältlich ist), so ist - bei einer Einsatzkonzentration dieser "Rohstofflösung" von 8,0 Gew.-% - in der Rezeptur nur einem "Aktivgehalt" von 4,0 Gew.-%, bezogen auf das Gesamtgewicht der Zubereitung, verwirklicht.

Daher war eine Aufgabe der vorliegenden Erfindung, kosmetische bzw. dermatologische Emulsionen mit hervorragenden hautpflegenden Eigenschaften zur Verfügung zu stellen.

Eine weitere Aufgabe der vorliegenden Erfindung war es, kosmetische oder dermatologische Emulsionen, insbesondere O/W-Emulsionen, zu finden, welche gegenüber erhöhten Elektrolytkonzentrationen stabil sind.

Femer war eine Aufgabe der vorliegenden Erfindung, Zubereitungen zur Verfügung zu stellen, welche den Zustand der Haut deutlich verbessern und insbesondere die Hautrauhigkeit vermindern.

Es hat sich überraschend gezeigt, und darin liegt die Lösung dieser Aufgaben, daß kosmetische und dermatologische Emulsionen mit mindestens einer wässrigen Phase, die

a) mindestens einen hydrophilen Emulgator A, gewählt aus der Gruppe der ethoxylierten Emulgatoren mit einem Ethoxylierungsgrad von 20 bis 100,
b) mindestens einen hydrophoben Emulgator B, gewählt aus der Gruppe, die gebildet wird aus ethoxylierten Emulgatoren mit einem Ethoxylierungsgrad von 1 bis 5 und Stearinsäurederivaten, sowie
c) mindestens einen Fettalkohol, gewählt aus der Gruppe der verzweigten oder unverzweigten, gesättigten oder ungesättigten, aliphatischen Fettalkohole mit einer Kettenlänge von $C_{14}$ bis $C_{22}$, enthalten,

wobei das Verhältnis von Emulgator A zu Emulgator B zu Fettalkohol aus dem Bereich 2 : 3 : 5 bis 2 : 5 : 3 gewählt wird, den Nachteilen des Standes der Technik abhelfen.

Es war für den Fachmann nicht vorauszusehen gewesen, daß die erfindungsgemäßen Zubereitungen

- besser als feuchtigkeitsspendende Zubereitungen wirken,
- besser die Hautglättung fördern,
- sich durch besser Pflegewirkung auszeichnen,
- besser als Vehikel für kosmetische und medizinisch-dermatologische Wirkstoffe dienen
- höhere Stabilität gegenüber Zerfall in Öl- und Wasserphasen aufweisen und
- sich durch bessere Bioverträglichkeit auszeichnen würden

als die Zubereitungen des Standes der Technik.

Erstaunlicherweise sind die erfindungsgemäßen Emulsionen trotz Anwesenheit höherer Mengen an Elektrolyten - wie z. B. 5 Gew.-% Natriumchlorid, 8 Gew.-% Milchsäure-Natriumlactat bzw. 8 Gew.-% Phenylbenzimidazolsulfonsäure, jeweils bezogen auf das Gesamtgewicht der Zubereitungen - über einen langen Zeitraum selbst bei unterschiedlichen Lagertemperaturen sehr stabil und zeigen dabei eine sehr gute Hautverträglichkeit bei guten sensorischen Eigenschaften.

Vorteilhafte hydrophile Emulgatoren A im Sinne der vorliegenden Erfindung sind PEG-100-Stearate (Arlacel 165 von ICI), Polysorbat 85, PEG-25-Glyceryltrioleat, PEG-40-Sorbitanlanolat, Choleth-24 und Ceteth-24 (Solulan C-24 von Amerchol), PEG-40 Hydrogenated Castor Oil, PEG-20-Glyceryloleat, PEG-20-Stearat, Polysorbate 80, PEG-20-Methylglucosesesquistearat, PEG-30-Glycerylisostearat, PEG-20-Glyceryllaurat, Ceteth-20, Ceteareth-25, PEG-30-Stearat, PEG-30-Glycerylstearat, Polysorbat-20, Laureth-23, PEG-40-Stearat, PEG-30-Glyceryllaurat, PEG-50-Stearat, PEG-100-Stearat, PEG-150-Laurat, Ceteareth-20, Ceteareth-30 und Steareth-21, PEG-25 Hydrogenated Castor Oil, PEG-54 Hydrogenated Castor Oil, Polysorbate 60, PEG-40-Sorbitanperoleat, PEG-22-Dodecylglykolcopolymer, Steareth-20, Isosteareth-20, PEG-45-/Dodecylglykolcopolymer, Methoxy PEG-22-/Dodecylglykolcopolymer, Hydrogenated Coco Glycerides, PEG-40-Sorbitanperoleat, PEG-40-Sorbitanperisostearat, PEG-20-Glycerylstearat, PEG-20 Methylglucosesesquistearat, Oleth-20, Isoceteth-20 sowie PEG-20-Stearat und PEG-30-Stearat.

Besonders vorteilhafte hydrophile Emulgatoren A im Sinne der vorliegenden Erfindung sind beispielsweise PEG-100 Stearate, PEG-40 Stearate, PEG-30 Stearate, Polysorbate 60 und Steareth-21.

Die Gesamtmenge an einem oder mehreren erfindungsgemäßen hydrophilen Emulgatoren A in den fertigen kosmetischen oder dermatologischen Zubereitungen wird vorteilhaft aus dem Bereich von 0,1 bis 5,0 Gew.-%, bevorzugt von 0,5 bis 2,5 Gew.-% gewählt, bezogen auf das Gesamtgewicht der Zubereitungen.

Die Emulgatoren können sowohl einzeln als auch in beliebigen Mischungen miteinander eingesetzt werden. Die Liste der genannten Emulgatoren, die im Sinne der vorliegenden Erfindung eingesetzt werden können, soll selbstverständlich nicht limitierend sein.

Vorteilhafte hydrophobe Emulgatoren B sind im Sinne der vorliegenden Erfindung Glycerylstearate, Polyglyceryl-2-Dipolyhydroxystearat, Glykoldistearat, Glykoldilaurat, Diethylenglykoldilaurat, Sorbitantrioleat, Glykololeat, Glyceryldilaurat, Sorbitanmonostearate, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, Sorbitanoleat, Sorbitanisostearat, Trioleylphosphat, Sorbitanstearat, PEG-7 Hydrogenated Castor Oil, Steareth-2, Oleth-2, PEG-5-Soyasterol, PEG-6 Sorbitan Beeswax, Ceteth-2, Glycerylstearat SE, Oleth-3, Sorbitanpalmitat, Polyglyceryl-2-PEG-4-Stearat, Laneth-5, Ceteth-3, Laureth-3, Oleth-5, Sorbitanlaurat, Laureth-4, PEG-4-Laurat, Triceteareth-4-Phosphat, Triceteareth-4 Phosphate und Sodium $C_{14-17}$ Alkyl Sec Sulfonat (Hostacerin CG von Hoechst), Trilaureth-4-Phosphat, Sucrosestearat, PEG-8-Oleat, Trioleth-8-Phosphat, Ceteareth-6, Lecithin, Laureth-4-Phosphat, Propylenglykolstearat SE, Glycerylstearat SE, PEG-6 Caprylic/Capric Glycerides, Glyceryloleat und Propylenglykol, PEG-9-Stearat, Glyceryllanolat, Ceteth-2, Glycerylmyristat, Glycerylisostearat und Polyglyceryl-3 Oleat, Glyceryllaurat, Laureth-4, Glycerinmonostearat, Ceteareth-3, Lanolinalkohol, Steareth-2, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Steareth-2 und PEG-8-Distearat, Methylglucosedioleat, Sorbitanoleat und PEG-2 Hydrogenated Castor Oil und Ozokerit und Hydrogenated Castor Oil, PEG-2 Hydrogenated Castor Oil, Ozokerit, Hydrogenated Castor Oil, Hydrogenated Coco Glycerides, Polyglyceryl-4-Isostearat, PEG-8-Beeswax, Polyglyceryl-2-Laurat, Stearamidopropyl-PG-dimoniumchloridphosphat, PEG-7 Hydrogenated Castor Oil, Glycerylisostearat, Polyglyceryl-3-Diisostearate und Cetylpalmitat.

Besonders vorteilhafte hydrophobe Emulgatoren B im Sinne der vorliegenden Erfindung sind beispielsweise Steareth-2 sowie Sorbitanstearat, welches unter der Handelsbezeichnung Arlacel® 60 bei der Fa. ICI / Unichema erhältlich ist, und Glycerylstearat, welches unter der Handelsbezeichnung Tegin M bei der Th. Goldschmidt AG erhältlich ist.

Die Gesamtmenge an einem oder mehreren erfindungsgemäßen hydrophoben Emulgatoren B in den fertigen kosmetischen oder dermatologischen Zubereitungen wird vorteilhaft aus dem Bereich von 0,1 bis 10,0 Gew.-%, bevorzugt von 1,0 bis 5,0 Gew.-% gewählt, bezogen auf das Gesamtgewicht der Zubereitungen.

Die Emulgatoren können sowohl einzeln als auch in beliebigen Mischungen miteinander eingesetzt werden. Die Liste der genannten Emulgatoren, die im Sinne der vorliegenden Erfindung eingesetzt werden können, soll selbstverständlich nicht limitierend sein.

Erfindungsgemäß bevorzugt werden der oder die Fettalkohole gewählt aus der Gruppe der verzweigten oder unverzweigten, gesättigten oder ungesättigten, aliphatischen Fettalkohole mit einer Kettenlänge von $C_{16}$ (Cetylalkohol) bis $C_{18}$ (Stearylalkohol). Ganz besonders vorteilhaft sind ferner Mischungen aus beiden Fettalkoholen (Cetearylalkohol).

Die Gesamtmenge an einem oder mehreren erfindungsgemäß verwendeten Fettalkoholen in den fertigen kosmetischen oder dermatologischen Zubereitungen wird vorteilhaft aus dem Bereich von 0,1 bis 10,0 Gew.-%, bevorzugt 1,0 bis 5,0 Gew.-%, besonders bevorzugt aus dem Bereich 1,5 bis 3,0 Gew.-% gewählt, bezogen auf das Gesamtgewicht der Zubereitungen.

Es ist erfindungsgemäß vorteilhaft, die Gewichtsverhältnisse von hydrophilem zu hydrophobem Emulgator zu Fettalkohol aus dem Bereich von 2 : 3 : 5 bis 2 : 5 : 3, besonders bevorzugt wie 1 : 2 : 2 zu wählen.

Bevorzugt stellen die erfindungsgemäßen Emulsionen O/W-Emulsionen dar.

Erfindungsgemäß werden der oder die Elektrolyte vorteilhaft aus den folgenden Gruppen gewählt:

1) Wasserlösliche UV- Filtersubstanzen:

D. h. wasserlösliche, zumeist als Alkalisalze vorliegende UV-Filtersubstanzen, insbesondere solche, die an ihrem Molekülgerüst eine oder mehrere Sulfonsäuregruppen bzw. Sulfonatgruppen tragen.

Vorteilhafte wasserlösliche UV-Filtersubstanzen sind z. B.:

- Salze der 2-Phenylbenzimidazol-5-sulfonsäure, wie ihr Natrium-, Kalium- oder ihr Triethanolammonium-Salz, insbesondere das Phenylen-1,4-bis-(2-benzimidazyl)-3,3'-5,5'-tetrasulfonsäure-bis-natriumsalz sowie die Sulfonsäure selbst;
- Sulfonsäure-Derivate des 3-Benzylidencamphers, wie z. B. 4-(2-Oxo-3-bornylidenmethyl)benzolsulfonsäure und/oder 2-Methyl-5-(2-oxo-3-bornylidenmethyl)sulfonsäure und deren Salze, beispielsweise das entsprechende Natrium-, Kalium- oder Triethanolammonium-Salz,
- Sulfonsäure-Derivate von Benzophenonen, vorzugsweise 2-Hydroxy-4-methoxybenzophenon-5-sulfonsäure und ihre Salze, beispielsweise das entsprechende Natrium-. Kalium- oder Triethanolammonium-Salz,
- 1,4-di(2-oxo-10-sulfo-3-bornylidenmethyl-)Benzol und dessen Salze (die entsprechenden 10-Sulfatoverbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet, das sich durch die folgende Struktur auszeichnet:

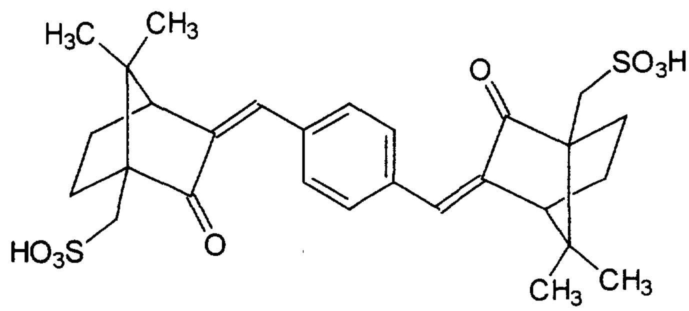

2) Salze mit folgenden Anionen:

Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.

Als Kationen dieser Salze werden bevorzugt Ammonium-, Alkylammonium-, Alkalimetall-, Erdalkalimetall-, Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

3) Aminosäuren und deren Salze bzw. deren Anionen:

Aminosäuren sind Bestandteil des natürlichen Feuchtigkeitsfaktors (der sogenannte Natural Moisturizing Factor). Der Zusatz von Aminosäuren, insbesondere essentieller Aminosäuren, ist als vorteilhaft anzusehen, da über Hydrataktionsvorgänge Feuchtigkeit in der Haut gebunden werden kann.

Aminosäuren mit besonders vorteilhafter kosmetischer bzw. dermatologischer Wirkung sind Glycin, Aladin, Valin, Leucin, Isoleucin, Phenylalaine, Tyrosin, Polin, Hydroxyprolin, Sein, Threonin, Cystein, Methionin, Tryptophan, Arginin.

Erfindungsgemäß werden der oder die Elektrolyte weiter vorteilhaft gewählt aus der Gruppe

4) der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,- Alkalimetall-, Erdalkalimetall,- Magnesium-, Eisen- bzw. Zinkionen.

α-Hydroxycarbonsäuren, welche kosmetisch oder dermatologisch relevant sind, folgen der allgemeinen Formel

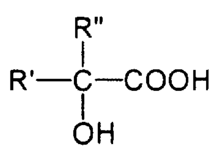

β-Hydroxycarbonsäuren, welche kosmetisch oder dermatologisch relevant sind, folgen der allgemeinen Formel

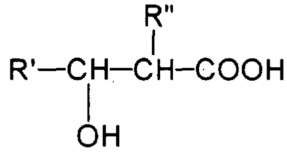

bzw.

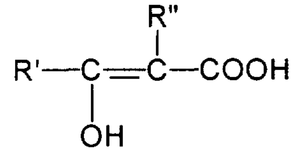

α-Ketocarbonsäuren, welche kosmetisch oder dermatologisch relevant sind, folgen der allgemeinen Formel

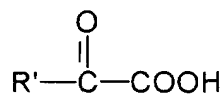

wobei jeweils R' und R" unabhängig voneinander gewählt werden aus der Gruppe

(a) H- ,
(b) verzweigtes oder unverzweigtes $C_{1-25}$-Alkyl-,
(c) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-
(d) Phenyl-,
(e) mit einer oder mehreren Carboxylgruppen und/oder Hydroxygruppen und/oder verzweigten und/oder unverzweigten $C_{1-25}$-Alkylgruppen substituiertes Phenyl-,
oder wobei das α-Kohlenstoffatom und das β-Kohlenstoffatom der β-Hydroxycarbonsäure mit R' und R" zusammen eine
(f) unsubstituierte Cycloalkylgruppe oder Arylgruppe mit 3 bis 7 Ringatomen oder eine
(g) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen
ausbildet und
(h) wobei die α-Hydroxycarbonsäuren oder die β-Hydroxycarbonsäuren oder die α-Ketocarbonsäuren gegebenenfalls in Form ihrer physiologisch verträglichen Salze vorliegen können.

Es folgen vorteilhaft im Rahmen der vorliegenden Erfindung zu verwendende α-Hydroxycarbonsäuren, β-Hydroxycarbonsäuren und α-Ketocarbonsäuren, wobei diese auch stellvertretend für ihre Salze bzw. Anionen aufgeführt werden:
Salicylsäure (auch 2-Hydroxybenzoësäure, Spirsäure), welche durch die Struktur

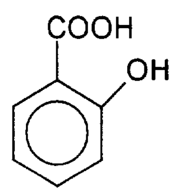

gekennzeichnet ist. Bekanntermaßen wirkt Salicylsäure antibakteriell und keratolytisch und ist Bestandteil mancher kosmetischen oder pharmazeutischen Zubereitungen.
Die den erfindungsgemäß verwendeten α-Hydroxycarbonsäuren werden vorteilhaft gewählt aus folgenden Substanzklassen:

(I) α-Hydroxyfettsäuren, wobei diese wiederum besonders vorteilhaft aus der Gruppe der $C_{10-18}$-Alkylcarbonsäuren gewählt werden,
(II) α-Hydroxyzuckersäuren, aliphatische α-Hydroxyfruchtsäuren,
(III) unsubstituierte aromatische α-Hydroxycarbonsäuren (z.B. Mandelsäure) bzw.
(IV) substituierte aromatische α-Hydroxycarbonsäuren.

Die unter Punkt (I) fallenden α-Hydroxyfettsäuren werden besonders vorteilhaft gewählt aus der Gruppe

■ α-Hydroxycarbonsäuren, gemäß der Formel

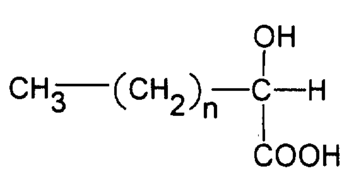

und/oder

- α-Hydroxy-isocarbonsäuren, gemäß der Formel

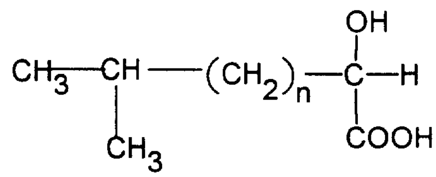

und/oder

- α-Hydroxy-anteisocarbonsäuren, gemäß der Formel

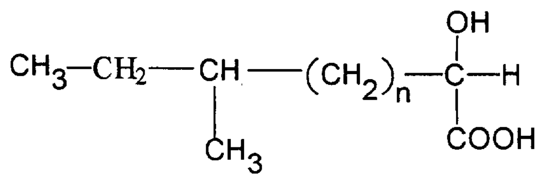

,

wobei n jeweils eine Zahl von 7 bis 31 darstellt.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (II) fallenden α-Hydroxyzuckersäuren werden besonders vorteilhaft gewählt aus der Gruppe der

- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (II) fallenden aliphatischen α-Hydroxyfruchtsäuren werden besonders vorteilhaft gewählt aus der Gruppe Äpfelsäure, Milchsäure, Citronensäure, Weinsäure (Dihydroxybernsteinsäure).

Bekanntermaßen wird Citronensäure zur Pufferung kosmetischer und/oder dermatologischer Zubereitungen, aber auch als Synergist für Antioxidantien in der Haut- und Haarkosmetik verwendet.

Bevorzugte α-Ketocarbonsäure ist die Brenztraubensäure (α-Oxopropansäure). Sie zeichnet sich durch folgende Struktur aus:

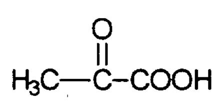

Vorteilhafter Elektrolyt ist ferner Harnstoff, insbesondere auch in Kombination mit Natriumlactat und/oder Milchsäure bzw. Natriumcitrat und/oder Citronensäure.

Die Höchstmenge der einzusetzenden Elektrolyte ist letztendlich abhängig von deren Löslichlichkeit in der wässrigen Phase. Grundsätzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolyts hinausgehende zusätzliche Menge dieses Elektrolyts, beispielsweise als ungelösten Festkörper, in eine kosmetische oder dermatologische Zubereitung einzuarbeiten.

Die erfindungsgemäßen Zubereitungen können darüber hinaus auch Hydrokolloide enthalten, beispielsweise um die Viskosität einzustellen.

Vorteilhafte Hydrokolloide sind z. B.:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind ferner beispielsweise Methylcellulosen, als welche die Methylether der Cellulose bezeichnet werden. Sie zeichnen sich durch die folgende Strukturformel aus

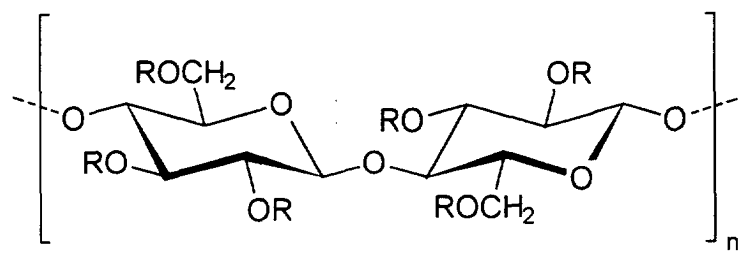

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das NatriumSalz des Glykolsäureethers der Cellulose, für welches R in Strukturformel I ein Wasserstoff und/oder $CH_2$-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen ("repeated units") besteht aus Glucose, Mannose, Glucuronsäure, Acetat und Pyruvat.

Erfindungsgemäß vorteilhafte Hydrokolloide sind ferner Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Carbopole sind Verbindungen der allgemeinen Strukturformel

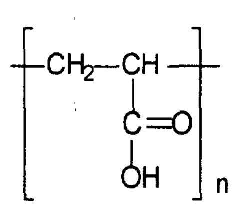

,

deren Molgewicht zwischen ca. 400 000 und mehr als 4 000 000 betragen kann. In die Gruppe der Carbopole gehören ferner Acrylat-Alkylacrylat-Copolymere, beispielsweise solche, die sich durch die folgende Struktur auszeichnen:

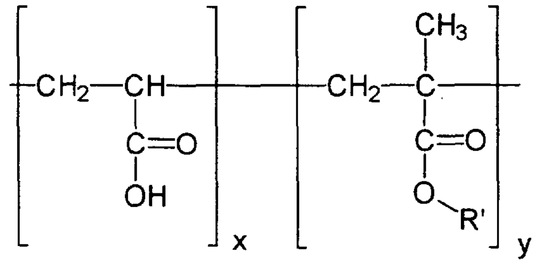

Darin stellen R' einen langkettigen Alkylrest und x und y Zahlen dar, welche den jeweiligen stöchiometrischen Anteil der jeweiligen Comonomere symbolisieren. Auch diese Carbopole sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984, wobei diese Verbindungen einzeln oder in beliebigen Kombinationen untereinander vorliegen können. Besonders bevorzugt sind Carbopol 981, 1382 und 5984 (sowohl einzeln als auch in Kombination mit weiteren Hydrokolloiden).

Ferner vorteilhaft im Sinne der vorliegenden Erfindung sind die den Acrylat-Alkylacrylat-Copolymeren vergleichbaren Copolymere aus $C_{10-30}$-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Die Gesamtmenge an einem oder mehreren Hydrokolloiden wird in den fertigen kosmetischen oder dermatologischen Emulsionen vorteilhaft kleiner als 1,0 Gew.-%, bevorzugt zwischen 0,01 und 0,5 Gew.-%, bezogen auf das Gesamtgewicht der Zubereitungen gewählt.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\ Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel® 1000 von der Gesellschaft SOLABIA S.A. erhältlich ist.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-Acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Stiftzubereitungen mit einem Gehalt an einem oder mehreren Antioxidantien zu versehen, wenn die Zubereitungen einem anderen Zweck dienen sollen, z. B. als Desodorantien oder Sonnenschutzmittel.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Die Liste der genannten Wirkstoffe bzw. Wirkstoffkombinationen, die in den erfindungsgemäßen Emulsionen verwendet werden können, soll selbstverständlich nicht limitierend sein.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Femer kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz sowie das 1,4-di(2-oxo-10-Sulfo-3-bomylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate. Insbesondere bevorzugt sind das 2,4-Bis-{[4-(2-Ethyl-hexyloxy)-2-hydroxy]-phenyl}-6-(4-methoxyphenyl)-13,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Besonders bevorzugte UV-Filtersubstanz im Sinne der vorliegenden Erfindung ist ferner ein unsymmetrisch substituiertes s-Triazin, welches auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxypropyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B-Filter können öllöslich oder wasserlöslich sein. Vorteilhafte öllösliche UV-B-Filtersubstanzen sind z. B.:

- 3-Benzyiidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-Aund/oder UV-B-Filter in kosmetische oder dermatologische Zubereitungen einzuarbeiten, beispielsweise bestimmte Salicylsäurederivate wie 4-lsopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Emulsionen als Grundlage für kosmetische Desodorantien betrifft. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden. Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) und dergleichen mehr. Auch Natriumhydrogencarbonat ist vorteilhaft zu verwenden.

Die Liste der genannten Wirkstoffe bzw. Wirkstoffkombinationen, die in den erfindungsgemäßen Emulsionen

verwendet werden können, soll selbstverständlich nicht limitierend sein.

Die erfindungsgemäßen kosmetischen Desodorantien können in Form von aus normalen Behältern auftragbaren wasserhaltigen, kosmetischen Stifte vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Zubereitung.

Die erfindungsgemäßen Stifte sind ferner hervorragende Vehikel für dermatologische Wirkstoffe. Insbesondere eignen sie sich als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Daher ist es vorteilhaft, den erfindungsgemäßen Zubereitungen gegen Akne wirksame Substanzen zuzugeben, die beispielsweise gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol ($\alpha$-Octadecylglycerylether), Selachylalkohol ($\alpha$-9-Octadecenylglycerylether), Chimylalkohol ($\alpha$-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und CalciumSalze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen (6-[3-(1-Adamantyl)-4-methoxyphenyl]-2-naphthoesäure), Azelainsäure (Nonandisäure), Mesulfen (2,7--Dimethylthianthren, $C_{14}H_{12}S_2$) sowie Aluminiumoxid, Zinkoxid und/oder feinverteilter Schwefel.

Die Menge der Antiaknemittel (eine oder mehrere Verbindungen) in den Zubereitungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Zubereitung.

Die Herstellung der erfindungsgemäßen kosmetischen und/oder dermatologischen Zubereitungen erfolgt in der dem Fachmann üblichen Weise, zumeist dergestallt, daß eine Ölphase mit einem Gehalt an erfindungsgemäß verwendeten grenzflächenaktiven Glucosederivaten bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wäßrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1 (O/W-Emulsion):** | |
|---|---|
| | Gew.-% |
| PEG-100 Stearat | 1,20 |
| Sorbitanmonostearat | 2,40 |
| Cetylstearylalkohol | 2,40 |
| Paraffinum liquidum | 12,00 |
| Xanthangummi | 0,20 |
| Glycerin | 3,00 |
| Milchsäure (90 %ige Lösung) | 2,00 |
| Natriumlactat (50 %ige Lösung) | 14,00 |
| Parfüm, Konservierungsmittel, Farbstoffe, Antioxidantien usw. | q.s. |

(fortgesetzt)

| **Beispiel 1 (O/W-Emulsion):** | |
|---|---|
| | Gew.-% |
| Wasser | ad 100,00 |
| pH-Wert eingestellt auf | 5,0 |

| **Beispiel 2 (O/W-Emulsion):** | |
|---|---|
| | Gew.-% |
| PEG-40 Stearat | 1,20 |
| Sorbitanmonostearat | 2,70 |
| Cetylstearylalkohol | 2,30 |
| Octyldodecanol | 3,00 |
| Caprylic/Capric Triglyceride | 3,00 |
| Dicaprylylether | 3,00 |
| Xanthangummi | 0,10 |
| Hydroxypropyl Starch Phosphat | 0,20 |
| Glycerin | 5,00 |
| Milchsäure (90 %ige Lösung) | 0,50 |
| Natriumlactat (50 %ige Lösung) | 15,00 |
| Parfüm, Konservierungsmittel, NaOH Farbstoffe, Antioxidantien usw. | q.s. |
| Wasser | ad 100,00 |
| pH-Wert eingestellt auf | 5,0 |

| **Beispiel 3 (O/W-Emulsion):** | |
|---|---|
| | Gew.-% |
| PEG-100 Stearat | 1,20 |
| Glyceryl Stearat | 2,40 |
| Cetylstearylalkohol | 2,40 |
| Paraffinum liquidum | 14,00 |
| Dicaprylylether | 3,00 |
| Xanthangummi | 0,20 |
| Glycerin | 3,00 |
| Citronensäure | 0,20 |
| Natriumcitrat | 0,40 |
| Parfüm, Konservierungsmittel, Farbstoffe, Antioxidantien usw. | q.s. |
| Wasser | ad 100,00 |
| pH-Wert eingestellt auf | 5,0 |

| **Beispiel 4 (O/W-Emulsion):** | |
|---|---|
| | Gew.-% |
| PEG-100 Stearat | 1,20 |
| Glyceryl Stearat | 2,40 |
| Cetylstearylalkohol | 2,40 |
| Octyldodecanol | 3,00 |

(fortgesetzt)

| **Beispiel 4 (O/W-Emulsion):** | |
|---|---|
| | Gew.-% |
| Caprylic/Capric Triglyceride | 2,00 |
| Squalan | 5,00 |
| Jojoba Öl | 1,00 |
| Cyclomethicon | 3,00 |
| Dimethicon | 0,50 |
| Paraffinum liquidum | 2,00 |
| Hydrierte Kokosfettsäureglyceride | 2,00 |
| Xanthangummi | 0,10 |
| Glycerin | 10,00 |
| Natriumchlorid | 5,00 |
| Tocopherolacetat | 1,00 |
| Serin | 0,50% |
| Parfüm, Konservierungsmittel, Farbstoffe, Antioxidantien usw. | q.s. |
| Wasser | ad 100,00 |
| pH-Wert eingestellt auf | 6,0 |

| **Beispiel 5 (O/W-Emulsion):** | |
|---|---|
| | Gew.-% |
| Steareth-21 | 1,20 |
| Steareth-2 | 2,40 |
| Cetylstearylalkohol | 2,40 |
| Paraffinum liquidum | 15,00 |
| Xanthangummi | 0,15 |
| Glycerin | 5,50 |
| Milchsäure (90 %ige Lösung) | 1,50 |
| Natriumlactat (50% %ige Lösung) | 12,00 |
| Parfüm, Konservierungsmittel, Farbstoffe, Antioxidantien usw. | q.s. |
| Wasser | ad 100,00 |
| pH-Wert eingestellt auf | 5,0 |

| **Beispiel 6 (O/W-Emulsion):** | |
|---|---|
| | Gew.-% |
| PEG-30 Stearat | 1,20 |
| Glyceryl Stearat | 2,40 |
| Cetylalkohol | 2,40 |
| Octyldodecanol | 3,00 |
| Caprylic/Capric Triglyceride | 3,00 |
| Dicaprylylether | 3,00 |
| Xanthangummi | 0,15 |
| Glycerin | 3,00 |
| Natriumchlorid | 4,50 |
| Parfüm, Konservierungsmittel, Farbstoffe, Antioxidantien usw. | q.s. |

(fortgesetzt)

| Beispiel 6 (O/W-Emulsion): | |
|---|---|
| | Gew.-% |
| Wasser | ad 100,00 |
| pH-Wert eingestellt auf | 6,1 |

| Beispiel 7 (O/W-Emulsion): | |
|---|---|
| | Gew.-% |
| PEG-40 Stearat | 1,20 |
| Glyceryl Stearat | 2,40 |
| Myristylalkohol | 2,40 |
| Octyldodecanol | 3,00 |
| Caprylic/Capric Triglyceride | 3,00 |
| Dicaprylylether | 3,00 |
| Xanthangummi | 0,20 |
| Glycerin | 6,50 |
| Arginin Hydrochlorid | 1,20 |
| Parfüm, Konservierungsmittel, Farbstoffe, Antioxidantien usw. | q.s. |
| Wasser | ad 100,00 |
| pH-Wert eingestellt auf | 5,0 |

| Beispiel 8 (O/W-Emulsion): | |
|---|---|
| | Gew.-% |
| Polysorbate-60 | 1,20 |
| Glycerylmonostearat | 2,40 |
| Cetylalkohol | 1,00 |
| Behenylalkohol | 1,50 |
| Octyldodecanol | 3,00 |
| Caprylic/Capric Triglyceride | 3,00 |
| Dicaprylylether | 3,00 |
| hydogenated Polyisobuten | 6,00 |
| Xanthangummi | 0,25 |
| Glycerin | 9,00 |
| 2-Phenylbenzimidazol-5-sulfonsäure | 8,00 |
| Parfüm, Konservierungsmittel, NaOH Farbstoffe, Antioxidantien usw. | q.s. |
| Wasser | ad 100,00 |
| pH-Wert eingestellt auf | 7,0 |

| Beispiel 9 (O/W-Emulsion): | |
|---|---|
| | Gew.-% |
| PEG-100 Stearat | 0,90 |
| PEG-40 Stearat | 0,20 |
| PEG-30 Stearat | 0,15 |
| Glyceryl Stearat | 2,30 |

(fortgesetzt)

| **Beispiel 9 (O/W-Emulsion):** | |
|---|---|
| | Gew.-% |
| Cetylalkohol | 1,70 |
| Stearylalkohol | 0,60 |
| Octyldodecanol | 1,00 |
| Caprylic/Capric Triglyceride | 2,00 |
| Dicaprylylether | 2,00 |
| Paraffinum liquidum | 9,00 |
| Xanthangummi | 0,10 |
| Glycerin | 5,00 |
| Milchsäure (90 %ige Lösung) | 0,12 |
| Natriumlactat (50 %ige Lösung) | 8,00 |
| Harnstoff | 4,00 |
| Octylmethoxycinnamat | 4,00 |
| Benzophenone-3 | 3,00 |
| Octylsalicylat | 3,00 |
| Parfüm, Konservierungsmittel Farbstoffe, Antioxidantien usw. | q.s. |
| Wasser | ad 100,00 |
| pH-Wert eingestellt auf | 5,0 |

| **Beispiel 10 (Emulgatorgel):** | |
|---|---|
| | Gew.-% |
| PEG-100 Stearat | 1,20 |
| Glyceryl Stearat | 2,40 |
| Cetylstearylalkohol | 2,40 |
| Xanthangummi | 0,10 |
| Glycerin | 4,00 |
| Milchsäure (90 %ige Lösung) | 0,50 |
| Natriumlactat (50 %ige Lösung) | 15,00 |
| Parfüm, Konservierungsmittel, Farbstoffe, Antioxidantien usw. | q.s. |
| Wasser | ad 100,00 |
| pH-Wert eingestellt auf | 5,0 |

| **Beispiel 11 (Deoemulsion):** | |
|---|---|
| | Gew.-% |
| PEG-100 Stearat | 1,20 |
| Glyceryl Stearat | 2,40 |
| Cetylstearylalkohol | 2,40 |
| Xanthangummi | 0,21 |
| Glycerin | 3,00 |
| Ethanol | 10,00 |
| Aluminiumchlorohydrat (50 %ige Lösung) | 15,00 |
| Parfüm, Konservierungsmittel, Farbstoffe, Antioxidantien usw. | q.s. |
| Wasser | ad 100,00 |

(fortgesetzt)

| Beispiel 11 (Deoemulsion): | |
|---|---|
| | Gew.-% |
| pH-Wert eingestellt auf | 4,4 |

**Patentansprüche**

**1.** Kosmetische und dermatologische Emulsionen mit mindestens einer wässrigen Phase, die

a) mindestens einen hydrophilen Emulgator A, gewählt aus der Gruppe der ethoxylierten Emulgatoren mit einem Ethoxylierungsgrad von 20 bis 100,
b) mindestens einen hydrophoben Emulgator B, gewählt aus der Gruppe, die gebildet wird aus ethoxylierten Emulgatoren mit einem Ethoxylierungsgrad von 1 bis 5 und Stearinsäurederivaten, sowie
c) mindestens einen Fettalkohol, gewählt aus der Gruppe der verzweigten oder unverzweigten, gesättigten oder ungesättigten, aliphatischen Fettalkohole mit einer Kettenlänge von $C_{14}$ bis $C_{22}$, enthalten,

wobei das Verhältnis von Emulgator A zu Emulgator B zu Fettalkohol aus dem Bereich 2 : 3 : 5 bis 2 : 5 : 3 gewählt wird.

**2.** Verwendung von kosmetischen und dermatologischen Emulsionen, insbesondere O/W-Emulsionen mit mindestens einer wässrigen Phase, die

a) mindestens einen hydrophilen Emulgator A, gewählt aus der Gruppe der ethoxylierten Emulgatoren mit einem Ethoxylierungsgrad von 20 bis 100,
b) mindestens einen hydrophoben Emulgator B, gewählt aus der Gruppe, die gebildet wird aus ethoxylierten Emulgatoren mit einem Ethoxylierungsgrad von 1 bis 5 und Stearinsäurederivaten, sowie
c) mindestens einen Fettalkohol, gewählt aus der Gruppe der verzweigten oder unverzweigten, gesättigten oder ungesättigten, aliphatischen Fettalkohole mit einer Kettenlänge von $C_{14}$ bis $C_{22}$, enthalten,

wobei das Verhältnis von Emulgator A zu Emulgator B zu Fettalkohol aus dem Bereich 2 : 3 : 5 bis 2 : 5 : 3 gewählt wird, zur Pflege der Haut.

**3.** Verwendung der Kombination aus

a) mindestens einen hydrophilen Emulgator A, gewählt aus der Gruppe der ethoxylierten Emulgatoren mit einem Ethoxylierungsgrad von 20 bis 100,
b) mindestens einen hydrophoben Emulgator B, gewählt aus der Gruppe, die gebildet wird aus ethoxylierten Emulgatoren mit einem Ethoxylierungsgrad von 1 bis 5 und Stearinsäurederivaten, sowie
c) mindestens einen Fettalkohol, gewählt aus der Gruppe der verzweigten oder unverzweigten, gesättigten oder ungesättigten, aliphatischen Fettalkohole mit einer Kettenlänge von $C_{14}$ bis $C_{22}$, enthalten,

wobei das Verhältnis von Emulgator A zu Emulgator B zu Fettalkohol aus dem Bereich 2 : 3 : 5 bis 2 : 5: 3 gewählt wird,
zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten.

**4.** Verwendung nach Anspruch 3 zum Erzielen oder Erhöhen der Stabilität von Emulsionen gegenüber der Gegenwart von Elektrolyten, insbesondere zum Erzielen oder Erhöhen der Stabilität von O/W-Emulsionen gegenüber der Gegenwart von Elektrolyten, wobei die Emulsionen in mindestens einer der wässrigen Phasen einen oder mehrere Elektrolyte gelöst enthält und die Gesamtmenge an dem oder den Elektrolyten größer als 5,0 Gew.-% gewählt wird, bezogen auf das Gesamtgewicht der Zubereitungen.

**5.** Zubereitung nach Anspruch 1 oder Verwendung nach einem der Ansprüche 2 bis 4, **dadurch gekennzeichnet, daß** die Gesamtmenge an einem oder mehreren hydrophilen Emulgatoren A in den fertigen kosmetischen oder dermatologischen Zubereitungen aus dem Bereich von 0,1 bis 5,0 Gew.-%, bevorzugt von 0,5 bis 2,5 Gew.-%

gewählt wird, bezogen auf das Gesamtgewicht der Zubereitungen.

**6.** Zubereitung nach Anspruch 1 oder Verwendung nach einem der Ansprüche 2 bis 4, **dadurch gekennzeichnet, daß** die Gesamtmenge an einem oder mehreren hydrophoben Emulgatoren B in den fertigen kosmetischen oder dermatologischen Zubereitungen aus dem Bereich von 0,1 bis 10,0 Gew.-%, bevorzugt von 1,0 bis 5,0 Gew.-% gewählt wird, bezogen auf das Gesamtgewicht der Zubereitungen.

**7.** Zubereitung nach Anspruch 1 oder Verwendung nach einem der Ansprüche 2 bis 4, **dadurch gekennzeichnet, daß** die Gesamtmenge an einem oder mehreren Fettalkoholen in den fertigen kosmetischen oder dermatologischen Zubereitungen gewählt wird aus dem Bereich von 0,1 von 10,0 Gew.-%, bevorzugt von 1,0 bis 5,0 Gew.-%, bezogen auf das Gesamtgewicht der Zubereitungen.

**8.** Zubereitung nach Anspruch 1 oder Verwendung nach einem der Ansprüche 2 bis 4, **dadurch gekennzeichnet, daß** die Gewichtsverhältnisse von hydrophilem Emulgator A zu hydrophobem Emulgator B zu Fettalkohol aus dem Bereich wie 1 : 2 : 2 gewählt werden.

Europäisches Patentamt

# EUROPÄISCHER RECHERCHENBERICHT

Nummer der Anmeldung
EP 01 10 9642

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | EP 1 072 246 A (BEIERSDORF AG) 31. Januar 2001 (2001-01-31) * Seite 2, Zeile 3 - Zeile 6; Beispiele 2,5-8 * * Seite 3, Zeile 18 - Zeile 42 * | 1-8 | A61K7/48 |
| X | EP 1 072 247 A (BEIERSDORF AG) 31. Januar 2001 (2001-01-31) * Beispiele 2,5-8 * | 1-8 | |
| X | EP 1 072 248 A (BEIERSDORF AG) 31. Januar 2001 (2001-01-31) * Beispiele 2,5-8 * | 1-8 | |
| X | DE 198 39 402 A (BEIERSDORF AG) 2. März 2000 (2000-03-02) * Spalte 2, Zeile 20 - Zeile 64 * * Beispiele 1,4 * * Spalte 3, Zeile 61 - Zeile 63 * | 1,2,5-8 | |
| X | FR 2 715 845 A (OREAL) 11. August 1995 (1995-08-11) * Beispiel 1 * | 1-8 | RECHERCHIERTE SACHGEBIETE (Int.Cl.7) A61K |
| A | US 5 750 124 A (GOHLA SVEN ET AL) 12. Mai 1998 (1998-05-12) * Spalte 8, Zeile 10 - Zeile 44; Beispiele * | 1-8 | |

Der vorliegende Recherchenbericht wurde für alle Patentansprüche erstellt

| Recherchenort | Abschlußdatum der Recherche | Prüfer |
|---|---|---|
| MÜNCHEN | 11. September 2001 | Pregetter, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 01 10 9642

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-09-2001

| Im Recherchenbericht angeführtes Patentdokument | Datum der Veröffentlichung | Mitglied(er) der Patentfamilie | Datum der Veröffentlichung |
|---|---|---|---|
| EP 1072246 A | 31-01-2001 | DE 19934945 A | 01-02-2001 |
| EP 1072247 A | 31-01-2001 | DE 19934946 A | 01-02-2001 |
| EP 1072248 A | 31-01-2001 | DE 19934944 A | 01-02-2001 |
| DE 19839402 A | 02-03-2000 | EP 0995428 A | 26-04-2000 |
| FR 2715845 A | 11-08-1995 | JP 2595483 B | 02-04-1997 |
| | | JP 7258064 A | 09-10-1995 |
| | | US 5560916 A | 01-10-1996 |
| US 5750124 A | 12-05-1998 | DE 4343833 A | 29-06-1995 |
| | | AT 175863 T | 15-02-1999 |
| | | AU 7738694 A | 10-07-1995 |
| | | WO 9517155 A | 29-06-1995 |
| | | DE 59407714 D | 04-03-1999 |
| | | EP 0735853 A | 09-10-1996 |
| | | ES 2128583 T | 16-05-1999 |
| | | JP 9506871 T | 08-07-1997 |

EPO FORM P0461

Für nähere Einzelheiten zu diesem Anhang : siehe Amtsblatt des Europäischen Patentamts, Nr.12/82